# FASCICULE DE BREVET EUROPEEN

(11) **EP 4 106 611 B1**
(45) Date de publication et mention de la délivrance du brevet: **24.07.2024**
(21) Numéro de dépôt: 21710542.8
(22) Date de dépôt: 18.02.2021
(51) Int. Cl.: A61B 3/10, A61B 3/11, A61B 3/00

(54) **PROCEDE DE MESURE D'UN MENISQUE LACRYMAL**
VERFAHREN ZUR MESSUNG EINES TRÄNENMENISKUS
METHOD FOR MEASURING A TEAR MENISCUS

(30) Priorité: 21.02.2020 FR 2001763
(43) Date de publication de la demande: 28.12.2022
(73) Titulaire: E-Swin Developpement, 78550 Houdan (FR)
(72) Inventeur: BROTTIER, Yves-Vincent, 78113 ADAINVILLE (FR); OBIN, Arnaud, 78660 PARAY-DOUAVILLE (FR); PERRIN, Nelson, 78730 SAINTE-MESME (FR)
(74) Mandataire: Plasseraud IP
(86) Numéro de dépôt international: PCT/FR2021/050285
(87) Numéro de publication internationale: WO 2021/165619

(56) Documents cités:
- WO-A2-2013/166477
- US-A1- 2005 200 707
- US-B1- 6 299 305
- US-B2- 9 078 599

## Description

La présente description fait référence à la demande FR 19 10129 déposée le 13 septembre 2019 au nom de la demanderesse auprès de L'INPI et à la demande de brevet FR19 10131 déposée le 13 septembre 2019 au nom de la demanderesse auprès de l'INPI.

### Domaine technique

L'invention relève du domaine des examens ophtalmologiques et notamment le contrôle de la lubrification oculaire et plus particulièrement la mesure de la hauteur du ménisque lacrymal.

### Technique antérieure

Le ménisque lacrymal est une accumulation de liquide lacrymal à l'interface entre la cornée et la paupière inférieure.

L'observation du ménisque lacrymal, ou tear meniscus en anglais fait partie des examens couramment réalisés par les praticiens dans le diagnostic différentiel du syndrome de l'œil sec.

Il est notamment intéressant d'évaluer la hauteur de ce ménisque: elle est représentative du volume lacrymal total.

Cette évaluation est classiquement réalisée en lumière blanche, sous lampe à fente ou à l'aide d'un système de visualisation / acquisition d'image avec caméra, objectif et éclairage et classiquement, le ménisque est interprété visuellement.

Le ménisque est essentiellement aqueux et transparent.

Dans l'observation en lumière blanche, la formation d'image met en oeuvre la superposition de deux phénomènes :
La visualisation de la structure existant derrière le ménisque lacrymal,
Une réflexion spéculaire de la source d'éclairage par le dioptre air-larme qui se comporte par définition comme un miroir et forme une ligne claire au niveau du ménisque. Le document US 9 078 599 B2 qui peut être considéré comme représentant l'état de la technique le plus proche, divulgue un procédé standard de mesure d'un ménisque lacrymal utilisant de l'imagerie angiographie à la fluorescéine en combinaison avec un "fluorescein angiography module" qui n'est configuré que pour créer des images de la surface du ménisque lacrymal, comme défini dans la préambule de la revendication 1.

### Problème technique

Le problème est que le ménisque lacrymal forme une surface courbe concave entre la cornée et la paupière ce qui fait que les rayons lumineux qui se réfléchissent sur la surface du ménisque proche de la paupière ne se dirigent pas tous vers un objectif placé face à l'œil ce qui rend la visualisation de certaines zones du ménisque délicate. En fait la réflexion spéculaire n'est possible que quand la tangente du ménisque est orientée de façon à renvoyer de la lumière dans la pupille de l'objectif. Il est clair que cela ne sera possible que sur une faible partie du ménisque. La ligne claire observée dans ce cas n'est donc pas représentative de la dimension du ménisque.

Les zones où l'on observe la structure de l'œil à travers le ménisque sont quant à elles difficiles à interpréter.

Ces solutions connues ne permettent donc pas de mesurer simplement la hauteur du ménisque lacrymal.

### Exposé de l'invention

Au vu de l'art antérieur, il est proposé un procédé de mesure d'un ménisque lacrymal utilisant de la fluorescéine se concentrant dans le ménisque lacrymal pour le rendre fluorescent, le procédé comprenant les opérations:
- d'instiller de la fluorescéine à la surface d'un oeil à examiner d'un patient,
- d'éclairer l'œil à examiner en lumière bleue et de capturer une image de l'œil à examiner, ladite image comportant des zones bleues non fluorescentes en absence de fluorescéine et des zones vertes fluorescentes en présence de fluorescéine,
et pour lequel l'analyse d'image comprend :
- une identification du ménisque lacrymal et une mesure de sa hauteur en pixels (h),
- une identification de l'iris et une mesure de son diamètre extérieur en pixels (d),
- le calcul d'un ratio (R= D/d) entre un diamètre physique extérieur (D) en millimètres de l'iris estimé ou mesuré par ailleurs et de sa mesure en pixel (d) et le calcul de la hauteur physique (H=Rxh) du ménisque lacrymal à partir de ce ratio.

Ce procédé est avantageux en ce qu'il permet une mesure rapide et fiable de la hauteur réelle totale du ménisque lacrymal.

Le diamètre physique extérieur estimé de l'iris peut être un diamètre basé sur une valeur moyenne associée au type d'oeil du patient. Pour ce faire, des tables de valeurs associées à des paramètres tels que la couleur des yeux, le type ou autre peuvent être intégrées dans un logiciel associé au procédé. Le diamètre physique extérieur de l'iris peut aussi être mesuré par ailleurs et sa valeur introduite dans un logiciel de calcul associé au procédé.

L'identification du ménisque lacrymal peut comporter une comparaison du ratio vert sur bleu des pixels de l'image avec un premier seuil déterminé.

Un seuil entre 0,8 et 1,3 et notamment de l'ordre de 0,95 est utilisable.

Le procédé peut comporter une opération d'exclusion des zones sombres adapté à éliminer les pixels pour lesquels le niveau de vert et/ou de bleu est inférieur à un second seuil déterminé.

Ceci permet d'éliminer des potentielles détections aberrantes sur des zones sombres telles que les niveaux vert et bleu tendant vers zéro le ratio vert/bleu devient indéfini.

Dans une forme de réalisation, le procédé comporte une segmentation de l'image identifiant les zones fluorescentes et plus particulièrement une transformation de l'image en image binaire affectant un premier niveau binaire au pixels majoritairement de couleur bleue et un second niveau binaire aux pixels majoritairement de couleur verte ou une classification des pixels de l'image selon un seuil sur un ratio vert/bleu.

L'image binaire permet d'identifier les zones fluorescentes et permet la détection du ménisque lacrymal.

Le procédé peut comporter un algorithme de recherche des composantes connexes et d'élimination des petits objets sur l'image transformée pour encore améliorer la détection du ménisque.

Le procédé peut comporter un algorithme d'application d'un opérateur de fermeture à l'image transformée en sorte d'éliminer les défauts locaux de petite taille sans déplacer les contours des zones d'un niveau binaire donné ce qui rend plus aisé l'étape suivante de calcul de polynôme de régression.

Un mode de réalisation particulièrement avantageux est l'utilisation d'un algorithme de calcul de polynômes de régression comportant :
- une détection et une mémorisation de segments verticaux perpendiculaire à la direction générale du ménisque lacrymal sur chaque colonne de l'image, lesdits segments comportant un début réalisé par une transition du premier niveau binaire vers le second niveau binaire et comportant une fin réalisée par une transition dudit second niveau vers ledit premier niveau, lesdits segments verticaux représentant le ménisque lacrymal fluorescent,
- à partir des transitions délimitant la partie supérieure du ménisque lacrymal constituée des débuts de segments correspondant à la transition de la zone non fluorescente vers la zone fluorescente, un calcul d'un premier polynôme de régression délimitant une ligne supérieure,
- à partir des transitions délimitant la partie inférieure du ménisque lacrymal constituée des fins de segments correspondant à la transition de la zone fluorescente vers la zone non fluorescente, un calcul d'un second polynôme de régression délimitant une ligne inférieure,

Le calcul du polynôme de régression peut utiliser la méthode des moindres carrés.

Le procédé peut comporter alors un calcul de la hauteur (h) du ménisque lacrymal en pixels réalisé en calculant la distance entre les polynômes de régression calculés pour chacune desdites première ligne et seconde ligne selon les colonnes de l'image entre la ligne supérieure et la ligne inférieure.

Selon un mode de réalisation permettant de simplifier la mesure, le procédé peut comporter après prise de l'image une sélection d'une zone d'intérêt autour de la paupière inférieure dudit oeil du patient pour réduire les temps de calcul et réduire les aléas de détection.

Selon la méthode d'instillation choisie, le procédé peut comporter une temporisation adaptée à attendre la résorption par écoulement dans les canaux lacrymaux du surplus de liquide lacrymal comportant ladite fluorescéine entre l'instillation de la fluorescéine et la mesure.

Si la fluorescéine est instillée sous forme de goutte, une à quelques minutes sont généralement suffisantes pour que l'excès de liquide introduit dans l'œil soit évacué par les canaux lacrymaux.

Il est visé en outre un programme informatique comportant des instructions pour la mise en oeuvre du procédé ci-avant lorsque ce programme est exécuté par un processeur.

Il est visé de plus un support d'enregistrement non transitoire lisible par un ordinateur sur lequel est enregistré un programme pour la mise en oeuvre du procédé ci-avant lorsque ce programme est exécuté par un processeur.

Il est visé en outre un dispositif de mesure ophtalmique adapté à mettre en oeuvre du procédé ci-avant et qui comporte un appareil de mesure ophtalmique pourvu de sources de lumière bleue disposées autour d'un objectif d'au moins une caméra, un calculateur pourvu d'une interface utilisateur et programmé pour piloter lesdites sources et ladite au moins une caméra et mettre en oeuvre le procédé, ledit calculateur et ladite interface étant intégrés dans le dispositif de mesure ophtalmique ou externes et reliés au dispositif de mesure ophtalmique.

### Brève description des dessins

D'autres caractéristiques, détails et avantages de l'invention apparaîtront à la lecture de la description détaillée ci-après, et à l'analyse des dessins annexés, sur lesquels :
[Fig. 1] montre un dispositif adapté à l'implémentation du procédé selon la description;
[Fig. 2] montre un détail d'une étape du procédé;
[Fig. 3A] montre une photo d'un oeil dans le cadre du procédé selon la description;
[Fig. 3B] montre une représentation schématique de la photo de la figure 3A;
[Fig. 4A] montre un détail de la figure 3A après sélection;
[Fig. 4B] montre une image binaire à partir de la figure 4A;
[Fig. 4C] montre une image d'une étape de génération de polynômes de régression;
[Fig. 5] est une vue schématique avec représentation des données mesurées ;
[Fig. 6] montre une vue schématique d'un procédé selon la description;
[Fig. 7] montre une vue schématique d'un procédé de mesure de diamètre extérieur d'iris applicable à l'invention.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

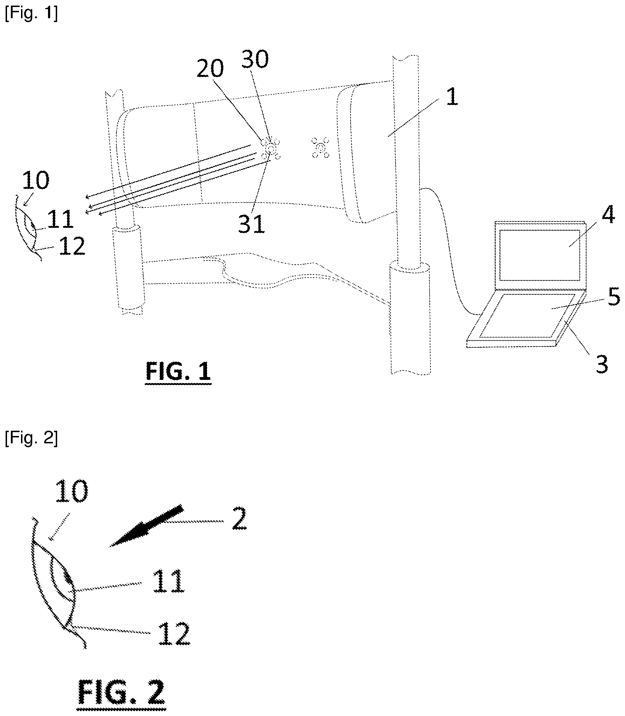

Il est maintenant fait référence à la figure 1 qui représente un dispositif adapté au procédé de l'invention et qui comporte un appareil de mesure ophtalmique 1 muni d'une ou deux caméras dont l'objectif 31 pointe vers la position des yeux du patient positionné devant l'appareil de mesure ophtalmique et muni de sources de lumière bleue 20 notamment à 470nm.

Selon l'exemple les sources de lumière sont quatre diodes électroluminescentes bleues appelées ci-après LEDs bleues selon l'acronyme de Light Emitting Diodes en anglais autour de l'objectif de chaque caméra.

Le dispositif comporte en outre un calculateur tel un ordinateur 3 comportant un écran 4, un clavier 5, pouvant être aussi une partie tactile de l'écran, une unité centrale processeur avec mémoire vive, morte et une mémoire de stockage de masse ainsi que les programmes nécessaires à la réalisation du procédé et une interface adaptée à piloter les sources de lumière et les caméras.

Le calculateur peut être externe ou directement intégré dans l'appareil de mesure ophtalmique, par exemple derrière les caméras comme décrit dans la demande de brevet FR19 10131 déposée le 13/09/2019 auprès de l'INPI.

Le procédé commence selon la figure 2 par une instillation de fluorescéine dans l'œil ou les deux yeux à examiner.

La fluorescéine est traditionnellement utilisée pour détecter les zones lésées de la conjonctive bulbaire ou de la cornée sur lesquelles elle se fixe temporairement. La rémanence est assez faible et la quantité fixée par la surface assez faible aussi.

La fluorescéine est miscible avec l'eau. Les larmes sont donc chargées de fluorescéine, et celle-ci se retrouve finalement dans le ménisque lacrymal ce qui rend le ménisque lacrymal fluorescent.

La fluorescéine peut être instillée en goutte et après une temporisation de quelques minutes, le volume liquide supplémentaire introduit par le goute de fluorescéine s'est évacué par les canaux lacrymaux. Il reste cependant suffisamment de fluorescéine pour marquer le volume lacrymal, qui devient alors fluorescent sous l'éclairage de la lumière bleue.

La fluorescéine peut aussi être instillée par tout autre moyen, par exemple au moyen de bandelettes et dans ce cas il n'y a pas a priori d'excédent de liquide et la mesure du procédé présenté ci-avant peut se faire sans temporisation.

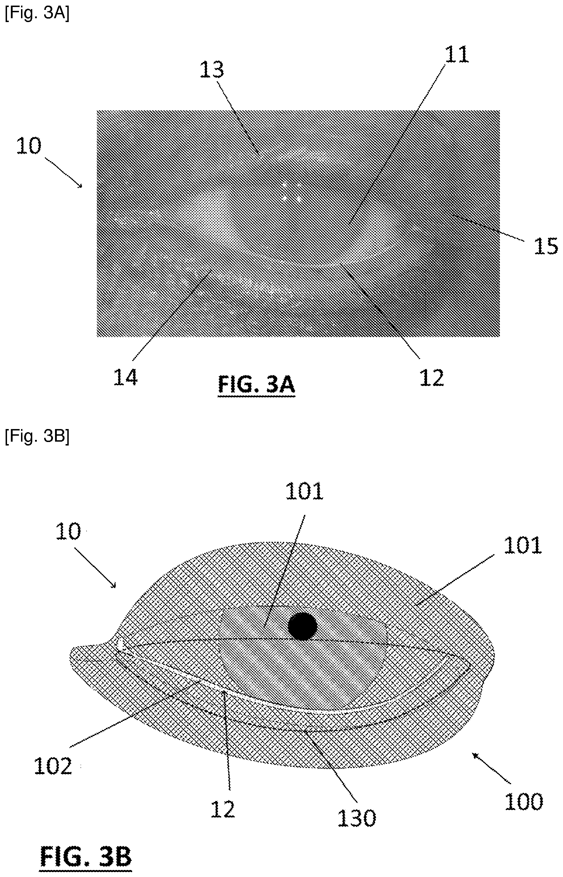

Une image de l'œil 10 après instillation vue sous lumière bleue est alors prise par la caméra qui lui correspond. L'image 100 vue en figure 3A comprend le ménisque lacrymal 12 fluorescent apparaissant en vert. Elle comprend en outre l'iris 11, la paupière supérieure 13 et la paupière inférieure 14 et des cils 15 qui rétrodiffusent la lumière incidente et apparaissent ainsi en bleu.

Selon la figure 3B schématisant l'image 100, le coloris bleu 101 est schématisé par des croisillons et le vert fluorescent 102 par un pointillé. Le ménisque lacrymal est ainsi très visible et bien délimité. Le contraste de couleur entre les zones vertes fluorescentes et les zones bleues non fluorescentes est élevé.

Dans une étape optionnelle, une zone d'intérêt 130 est tracée autour de la position du ménisque. Pour ce faire, le praticien entoure grossièrement la zone du ménisque lacrymal sur son écran. Ceci évite de faire les calculs de détection du ménisque lacrymal sur une zone aberrante qui pourrait être sélectionnée par le logiciel. Par exemple s'il y avait abondance de fluorescéine sur la paupière.

Une comparaison à un seuil sur le ratio vert/bleu permet d'identifier au moins en partie le ménisque lacrymal.

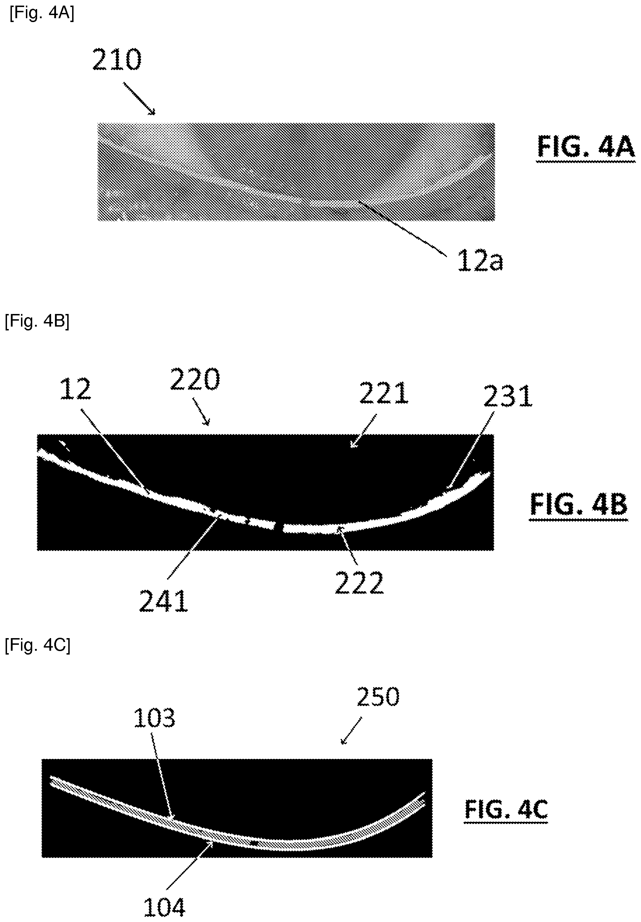

Dans l'image de la figure 3A, pour un pixel donné, le ratio vert/bleu est très significatif. En l'absence de fluorescéine, la lumière bleue incidente est rétrodiffusée. L'intensité du vert est très faible, voire nulle, le bleu est dominant. Le ratio vert/bleu tend vers 0 et en présence de fluorescéine, la lumière bleue est absorbée et réémise dans le vert. Le ratio vert/bleu est élevé, voire même tend vers l'infini dans la zone du ménisque lacrymal comme représenté en figure 4A pour laquelle la zone fluorescente 12a est lumineuse et de teinte saturée.

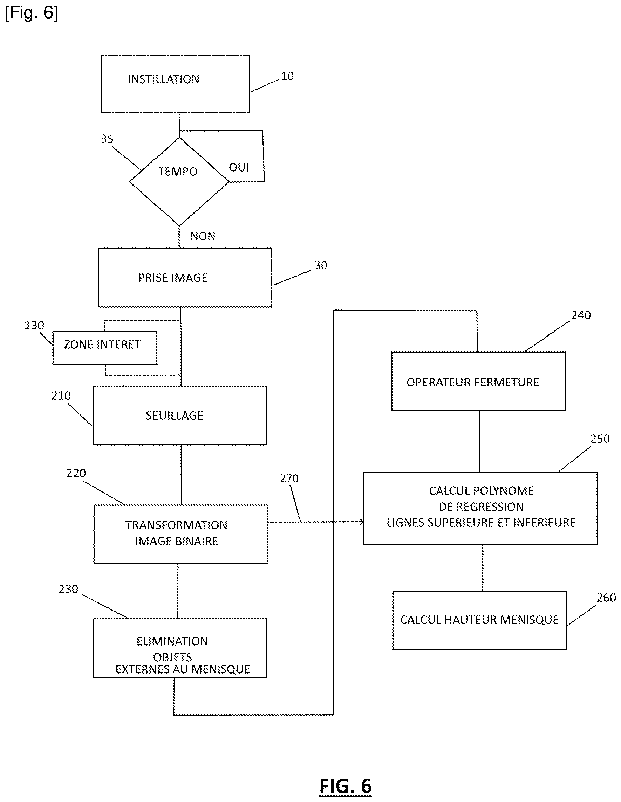

Le programme informatique du procédé schématisé à la figure 6 comporte ensuite un algorithme 220 qui va produire une image binaire de la zone à considérer et donner une valeur binaire 221 aux parties non fluorescentes et une valeur binaire 222 aux parties fluorescentes. Deux types de seuils sont utilisables :
En sensibilité: c'est le seuil sur le ratio vert/bleu

En exclusion de zones sombres: C'est un seuil permettant d'éliminer les pixels lorsque le maximum de vert et bleu est inférieur au seuil : le ratio vert / bleu est mal défini sur une image très sombre (c'est-à-dire vert et bleu de faible niveau). Typiquement sur les peaux sombres on pourrait détecter n'importe quoi sur la peau et donc détecter de la fluorescence là où il n'y en a pas.

Des valeurs de seuils sont proposées par défaut à l'opérateur qui peut par exemple les adapter en fonction du patient.

On obtient ainsi l'image binaire de la figure 48 où les points représentés en blanc sont les zones fluorescentes.

Dans une étape 230 suivante, le procédé comporte un algorithme de recherche des composantes connexes et d'élimination des petits objets 231 externes au ménisque sur l'image transformée. Les petits objets sont des pixels ou groupes de pixels dont la morphologie permet de déterminer qu'ils ne font pas partie du ménisque lacrymal et donc de les exclure des analyses d'image ultérieures. Il peut notamment exister des zones fluorescentes qui ne font pas partie du ménisque lacrymal, par exemple sur la conjonctive bulbaire ou sur les paupières et cet algorithme permet de les écarter du fait de leur morphologie (surface en pixels, rapport d'aspect largeur/hauteur ou autres caractéristiques).

Ensuite, le procédé peut comporter une étape 240 d'application à l'image binaire d'un opérateur de fermeture, qui permet de boucher des petits trous sans déplacer les contours. Par exemple cette étape permet de transformer en zones fluorescentes des zones non fluorescentes 241 dans une zone fluorescente de taille notablement supérieure. Cette opération est en particulier utile s'il y a une poussière, une bulle, ou un quelconque accident dans la zone fluorescente du ménisque lacrymal.

L'étape 250 comporte le calcul de polynômes de régression sur les contours supérieur et inférieur du ménisque lacrymal.

Pour ce faire, à partir de l'image binaire précédente on va définir des segments verticaux sur chaque colonne de l'image. Par convention on considère la direction verticale comme la direction perpendiculaire à une direction générale horizontale du ménisque lacrymal, ou la direction passant par les deux yeux du patient assis.

En considérant l'image binaire comme une image noir et blanc avec le blanc définissant la zone fluorescente, un segment vertical est défini par :

- Son début qui est la transition de noir vers blanc au début de la zone fluorescente;

- Sa fin qui est la transition de blanc vers noir en fin de la zone fluorescente.

L'ensemble des débuts de segment donne la partie supérieure de l'image binaire, c'est-à-dire la ligne de transition de la zone non fluorescente vers la zone fluorescente. L'ensemble des fins de segment donne la partie inférieure de l'image binaire, c'est-à-dire la ligne de transition de la zone fluorescente vers la zone non fluorescente.

Sur chacune des lignes supérieure et inférieure on calcule un polynôme de régression par la méthode des moindres carrés. Ce qui donne la ligne 103 pour le polynôme supérieur et la ligne 104 pour le polynôme inférieur sur la figure 4C. La régression polynomiale permet une résolution sub-pixel et permet de s'affranchir du bruit de quantification sur la position des transitions et aussi d'écarter des accidents locaux dans l'image : bruit, poussière, excroissance de la paupière par exemple. La distance entre le polynôme du haut et celui du bas est par principe nativement filtrée.

Les polynômes sont avantageusement d'ordre 4 pour suivre la courbure du ménisque.

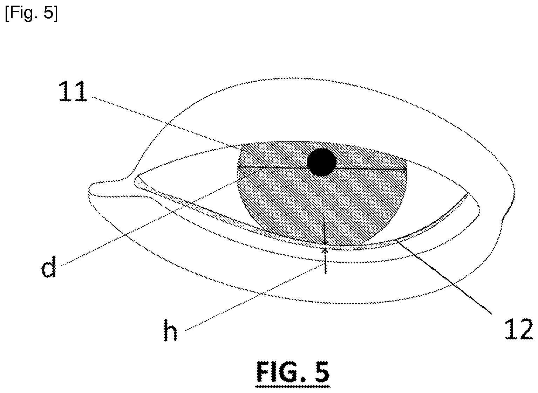

Ensuite, dans une étape 260, la distance entre les deux polynômes pour une colonne donnée de l'image est calculée pour donner la hauteur du ménisque lacrymal en pixels. Dans la figure 5 on a choisi comme position pour la mesure la verticale passant par le centre de l'iris, mais le praticien pourrait choisir d'autres points, éventuellement positionnés à sa demande.

Il est à noter que dans le cas où l'image ne comporterait pas d'objets de dimension inférieure à un seuil déterminé, les étapes d'élimination des petits objets 230 et d'application de l'opérateur de fermeture 240 pourraient être évitée pour passer directement de la génération de l'image binaire à l'étape de calcul des polynômes de régression comme décrit sur la figure 6 par la ligne pointillée 270.

Une seconde partie du procédé peut comprendre une détection automatique du diamètre extérieur de l'iris mesuré en pixels.

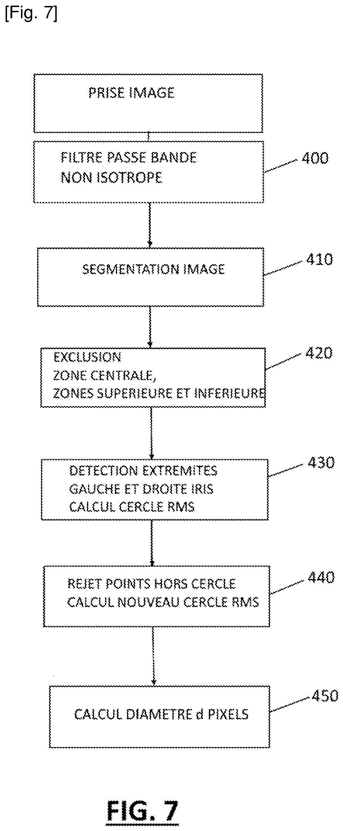

Le procédé comporte dans ce cas des étapes de détection d'iris tel que décrit dans la demande FR 19 10129 déposée le 13 septembre 2019 au nom de la demanderesse. Les étapes de détection d'iris peuvent comporter selon la figure 7 :
- une première transformation de l'image par l'application d'un filtre passe bande anisotrope 400 appliqué dans la direction de la largeur de l'œil pour produire des paires de transition montantes sombre vers clair et descendantes clair vers sombre selon un axe horizontal de l'œil ;
- une segmentation 410 de l'image pour rechercher les paires de transitions montante et descendante qui forment des segments devant être représentatifs de zones lumineuses dans l'image ;
- un filtrage de l'image 420 qui élimine les segments clairs de la zone centrale comportant le motif et des zones supérieure et inférieure de l'image ;
- la prise en compte des segments clairs, les autres zones de l'image n'étant plus considérées dans cette analyse, un premier calcul 430 d'un cercle de valeur efficace (ci-après valeur RMS selon l'acronyme de l'anglais root mean square, moyenne quadratique) du tour de l'iris à partir des extrémités droites des segments clairs à gauche de l'image et des extrémités gauches des segments clairs à droite de l'image;
- une étape 440 de suppression des points trop éloignés du cercle RMS ;
- pour les points restants, une nouvelle étape de calcul de cercle RMS pour suivre le contour de l'iris. A partir du cercle RMS, un calcul de diamètre extérieur de l'iris 450 par exemple par le calcul de la plus grande distance en pixels entre les bords du cercle va donner le diamètre extérieur d de l'iris en pixels ce qui permet en connaissant le diamètre extérieur réel de l'iris de calculer la hauteur réelle du ménisque lacrymal au moyen du calcul d'un ratio R= D/d entre le diamètre physique D de l'iris estimé ou mesuré par ailleurs et de sa mesure en pixel d et le calcul de la hauteur physique H=Rxh du ménisque lacrymal à partir de ce ratio.

L'invention ne se limite pas aux exemples décrits ci-avant mais elle englobe toutes les variantes que pourra envisager l'homme de l'art par exemple en modifiant l'ordre de certaines opérations, en en éliminant ou en ajoutant pour plus de rapidité de calcul ou plus de précision dans le cadre de la protection recherchée.

## Revendications

1. Procédé de mesure d'un ménisque lacrymal utilisant de la fluorescéine se concentrant dans le ménisque lacrymal pour le rendre fluorescent, à partir d'une analyse d'une image obtenue avec la mise en oeuvre d'opérations :
- d'instillation de la fluorescéine à la surface d'un oeil (10) à examiner d'un patient,
- d'éclairage de l'œil (10) à examiner en lumière bleue (20),
**caractérisé en ce qu'**il comprend :
- la capture (30) d'une image (100) de l'œil à examiner, ladite image comportant des zones bleues (101) non fluorescentes en absence de fluorescéine et des zones vertes (102) fluorescentes en présence de fluorescéine,
- une identification du ménisque lacrymal (12) et une mesure de sa hauteur en pixels (h),
- une identification de l'iris (11) et une mesure de son diamètre extérieur en pixels (d),
- le calcul d'un ratio (R= D/d) entre un diamètre extérieur physique (D) de l'iris estimé ou mesuré par ailleurs en millimètres et sa mesure en pixel (d) et le calcul de la hauteur physique (H=Rxh) du ménisque lacrymal à partir de ce ratio.

2. Procédé de mesure selon la revendication 1 pour lequel le diamètre extérieur physique estimé de l'iris est basé sur une valeur moyenne associée au type d'oeil du patient.

3. Procédé de mesure selon la revendication 1 ou 2 pour lequel l'identification du ménisque lacrymal (12) comporte une comparaison du ratio vert sur bleu des pixels de l'image avec un premier seuil déterminé.

4. Procédé de mesure selon la revendication 3 comportant une opération d'exclusion des zones sombres adapté à éliminer les pixels pour lesquels le niveau de vert et/ou de bleu est inférieur à un second seuil déterminé.

5. Procédé de mesure selon l'une quelconque des revendications précédentes comportant une transformation (220) de l'image en image binaire affectant un premier niveau binaire (221) au pixels majoritairement de couleur bleue et un second niveau binaire (222) aux pixels majoritairement de couleur verte ou une classification des pixels de l'image selon un seuil sur un ratio vert/bleu.

6. Procédé de mesure selon la revendication 5 comportant un algorithme (230) de recherche des composantes connexes et d'élimination des petits objets (231) sur l'image transformée.

7. Procédé de mesure selon la revendication 5 ou 6 comportant un algorithme (240) d'application d'un opérateur de fermeture à l'image transformée en sorte d'éliminer les défauts locaux de petite taille sans déplacer les contours des zones d'un niveau binaire donné.

8. Procédé de mesure selon la revendication 5, 6 ou 7 comportant un algorithme (250) de calcul de polynômes de régression comportant :
- une détection et une mémorisation de segments verticaux perpendiculaire à la direction générale du ménisque lacrymal sur chaque colonne de l'image, lesdits segments comportant un début réalisé par une transition du premier niveau binaire vers le second niveau binaire et comportant une fin réalisée par une transition dudit second niveau vers ledit premier niveau, lesdits segments verticaux représentant le ménisque lacrymal fluorescent,
- à partir des transitions délimitant la partie supérieure du ménisque lacrymal constituée des débuts de segments correspondant à la transition de la zone non fluorescente vers la zone fluorescente, un calcul d'un premier polynôme de régression délimitant une ligne supérieure (103),
- à partir des transitions délimitant la partie inférieure du ménisque lacrymal constituée des fins de segments correspondant à la transition de la zone fluorescente vers la zone non fluorescente, un calcul d'un second polynôme de régression délimitant une ligne inférieure (104),

9. Procédé de mesure selon la revendication 8 pour lequel le calcul du polynôme de régression utilise la méthode des moindres carrés.

10. Procédé de mesure selon la revendication 8 ou 9 comportant un calcul (260) de la hauteur (h) du ménisque lacrymal en pixels réalisé en calculant la distance entre les polynômes de régression calculés pour chacune desdites première ligne et seconde ligne selon les colonnes de l'image entre la ligne supérieure et la ligne inférieure.

11. Procédé de mesure selon l'une quelconque des revendications précédentes comportant une sélection d'une zone d'intérêt (130) autour de la paupière inférieure dudit oeil du patient pour réduire les temps de calcul et réduire les aléas de détection.

12. Procédé selon l'une quelconque des revendications précédentes comportant une temporisation (35) adaptée à attendre la résorption par écoulement dans les canaux lacrymaux du surplus de liquide lacrymal comportant ladite fluorescéine entre l'instillation de la fluorescéine et la mesure.

13. Programme informatique comportant des instructions pour la mise en oeuvre du procédé selon l'une des revendications 1 à 12 lorsque ce programme est exécuté par un processeur.

14. Support d'enregistrement non transitoire lisible par un ordinateur sur lequel est enregistré un programme pour la mise en oeuvre du procédé selon l'une des revendications 1 à 12 lorsque ce programme est exécuté par un processeur.

15. Dispositif de mesure ophtalmique pour la mise en oeuvre du procédé de l'une quelconque des revendications 1 à 12 **caractérisé en ce qu'**il comporte un appareil de mesure ophtalmique (1) pourvu de sources de lumière bleue (20) disposées autour d'un objectif (31) d'au moins une caméra (30), un calculateur pourvu d'une interface utilisateur (4, 5) et programmé pour piloter lesdites sources et ladite au moins une caméra et mettre en oeuvre le procédé, ledit calculateur et ladite interface étant intégrés dans le dispositif de mesure ophtalmique ou externes et reliés au dispositif de mesure ophtalmique.

## Patentansprüche

1. Verfahren zum Messen eines Tränenmeniskus unter Verwendung von Fluorescein, das sich in dem Tränenmeniskus konzentriert, um ihn fluoreszieren zu lassen, basierend auf einer Analyse eines Bildes, das durch die Durchführung der folgenden Maßnahmen erhalten wird:
- Instillation von Fluorescein auf die Oberfläche eines zu untersuchenden Auges (10) eines Patienten,
- Beleuchtung des zu untersuchenden Auges (10) mit blauem Licht (20),
**dadurch gekennzeichnet, dass** dies Folgendes umfasst:
- Erfassen (30) eines Bildes (100) des zu untersuchenden Auges, wobei das Bild bei Abwesenheit von Fluorescein nicht fluoreszierende blaue Bereiche (101) und bei Anwesenheit von Fluorescein fluoreszierende grüne Bereiche (102) aufweist,
- Identifizierung des Tränenmeniskus (12) und Messung seiner Höhe in Pixeln (h),
- Identifizierung der Iris (11) und Messung ihres Außendurchmessers in Pixeln (d),
- Berechnung eines Verhältnisses (R= D/d) zwischen einem geschätzten oder gemessenen physikalischen Außendurchmesser (D) der Iris in Millimetern und der Messung in Pixeln (d) sowie Berechnung der physikalischen Höhe (H=Rxh) des Tränenmeniskus aus diesem Verhältnis.

2. Messverfahren nach Anspruch 1, wobei der geschätzte physikalische Außendurchmesser der Iris auf einem Durchschnittswert beruht, der mit dem Augentyp des Patienten assoziiert ist.

3. Messverfahren nach Anspruch 1 oder 2, wobei die Identifizierung des Tränenmeniskus (12) einen Vergleich des Grün-Blau-Verhältnisses der Bildpixel mit einem ersten festgelegten Schwellenwert umfasst.

4. Messverfahren nach Anspruch 3, umfassend eine Maßnahme zum Ausschluss dunkler Bereiche, die dazu ausgebildet ist, Pixel zu eliminieren, bei denen das Grün- und/oder Blauverhältnis unter einem zweiten bestimmten Schwellenwert liegt.

5. Messverfahren nach einem der vorhergehenden Ansprüche, welches eine Transformation (220) des Bildes in ein binäres Bild umfasst, das ein erstes binäres Niveau (221) den Pixeln mit überwiegend blauer Farbe und ein zweites binäres Niveau (222) den Pixeln mit überwiegend grüner Farbe zuweist, oder eine Klassifizierung der Pixel des Bildes gemäß einem Schwellenwert auf einem Grün-Blau-Verhältnis.

6. Messverfahren nach Anspruch 5, umfassend einen Algorithmus (230) zur Suche nach zusammenhängenden Komponenten und zur Eliminierung kleiner Objekte (231) auf dem transformierten Bild.

7. Messverfahren nach Anspruch 5 oder 6, umfassend einen Algorithmus (240) zum Anwenden eines Schließoperators auf das transformierte Bild, um so kleine lokale Defekte zu entfernen, ohne die Konturen der Bereiche eines gegebenen binären Niveaus zu verschieben.

8. Messverfahren nach Anspruch 5, 6 oder 7, umfassend einen Algorithmus (250) zur Berechnung von Regressionspolynomen, umfassend:
- Erfassung und Speicherung von vertikalen Segmenten senkrecht zur allgemeinen Richtung des Tränenmeniskus in jeder Spalte des Bildes, wobei die Segmente einen Anfang umfassen, der durch einen Übergang von dem ersten binären Niveau zum zweiten binären Niveau realisiert wird, und ein Ende umfassen, das durch einen Übergang von dem zweiten Niveau zum ersten Niveau realisiert wird, wobei die vertikalen Segmente den fluoreszierenden Tränenmeniskus darstellen,
- ausgehend von den Übergängen, die den oberen Teil des Tränenmeniskus begrenzen, der aus den Anfängen der Segmente besteht, die dem Übergang von dem nicht fluoreszierenden Bereich zum fluoreszierenden Bereich entsprechen, Berechnung eines ersten Regressionspolynoms, das eine obere Linie (103) begrenzt,
- ausgehend von den Übergängen, die den unteren Teil des Tränenmeniskus begrenzen, der aus Segmentenden besteht, die dem Übergang von dem fluoreszierenden Bereich zu dem nicht fluoreszierenden Bereich entsprechen, Berechnung eines zweiten Regressionspolynoms, das eine untere Linie (104) abgrenzt.

9. Messverfahren nach Anspruch 8, wobei die Berechnung des Regressionspolynoms die Methode der kleinsten Quadrate verwendet.

10. Messverfahren nach Anspruch 8 oder 9, umfassend eine Berechnung (260) der Höhe (h) des Tränenmeniskus in Pixeln, die durch Berechnung des Abstands zwischen den Regressionspolynomen durchgeführt wird, die für jede der ersten Zeile und der zweiten Zeile gemäß den Spalten des Bildes zwischen der oberen Zeile und der unteren Zeile berechnet wurden.

11. Messverfahren nach einem der vorhergehenden Ansprüche, umfassend eine Auswahl eines betrachteten Bereichs (130) um das untere Augenlid des Auges des Patienten herum, um die Berechnungszeiten zu reduzieren und Zufälligkeiten bei der Erfassung zu verringern.

12. Verfahren nach einem der vorhergehenden Ansprüche, umfassend eine Zeitverzögerung (35), die dazu ausgebildet ist, die Resorption der überschüssigen, das Fluorescein enthaltenden Tränenflüssigkeit durch Abfließen in die Tränenkanäle zwischen der Instillation des Fluoresceins und der Messung abzuwarten.

13. Computerprogramm, umfassend Anweisungen zur Durchführung des Verfahrens nach einem der Ansprüche 1 bis 12, wenn dieses Programm von einem Prozessor ausgeführt wird.

14. Nicht-transientes Aufzeichnungsmedium, das von einem Computer lesbar ist, auf dem ein Programm zur Durchführung des Verfahrens nach einem der Ansprüche 1 bis 12 gespeichert ist, wenn dieses Programm von einem Prozessor ausgeführt wird.

15. Ophthalmische Messvorrichtung zur Durchführung des Verfahrens nach einem der Ansprüche 1 bis 12, **dadurch gekennzeichnet, dass** sie ein ophthalmisches Messgerät (1) umfasst, das mit Blaulichtquellen (20) versehen ist, die um ein Objektiv (31) wenigstens einer Kamera (30) herum angeordnet sind, einen Rechner, der mit einer Benutzerschnittstelle (4, 5) versehen und dazu programmiert ist, die Quellen und die wenigstens eine Kamera zu steuern und das Verfahren durchzuführen, wobei der Rechner und die Schnittstelle in das ophthalmische Messgerät integriert oder extern und mit dem ophthalmischen Messgerät verbunden sind.

## Claims

1. A method for measuring a tear meniscus using fluorescein concentrated in the tear meniscus to make it fluorescent, based on an analysis of an image obtained by implementing operations of:
- instilling fluorescein on the surface of a patient's eye (10) to be examined,
- illuminating the eye (10) to be examined with blue light (20),
**characterized in that** it comprises:
- capturing (30) an image (100) of the eye to be examined, said image comprising non-fluorescent blue regions (101) in the absence of fluorescein and fluorescent green regions (102) in the presence of fluorescein,
- identifying the tear meniscus (12) and measuring its height in pixels (h),
- identifying the iris (11) and measuring its outer diameter in pixels (d),
- calculating a ratio (R = D/d) of a physical outer diameter (D) of the iris as estimated or else measured in millimeters to its measurement in pixels (d) and calculating the physical height (H = Rxh) of the tear meniscus based on this ratio.

2. The measuring method as claimed in claim 1, wherein the estimated physical outer diameter of the iris is based on an average value associated with the patient's eye type.

3. The measuring method as claimed in claim 1 or 2, wherein identifying the tear meniscus (12) comprises comparing the green-to-blue ratio of the pixels of the image with a first determined threshold.

4. The measuring method as claimed in claim 3, comprising an operation of excluding dark regions which is designed to eliminate those pixels for which the green and/or blue level is lower than a second determined threshold.

5. The measuring method as claimed in any one of the preceding claims, comprising transforming (220) the image into a binary image assigning a first binary level (221) to those pixels which are predominantly blue in color and a second binary level (222) to those pixels which are predominantly green in color or classifying the pixels of the image according to a threshold with respect to a green/blue ratio.

6. The measuring method as claimed in claim 5, comprising an algorithm (230) for searching for connected components and eliminating small objects (231) in the transformed image.

7. The measuring method as claimed in claim 5 or 6, comprising an algorithm (240) for applying a closure operator to the transformed image so as to eliminate small local defects without moving the outlines of the regions of a given binary level.

8. The measuring method as claimed in claim 5, 6 or 7, comprising an algorithm (250) for calculating regression polynomials comprising:
- detecting and storing vertical segments perpendicular to the general direction of the tear meniscus in each column of the image, said segments comprising a start produced by a transition from the first binary level to the second binary level and comprising an end produced by a transition from said second level to said first level, said vertical segments representing the fluorescent tear meniscus,
- based on the transitions delimiting the upper portion of the tear meniscus made up of the starts of segments corresponding to the transition from the non-fluorescent region to the fluorescent region, calculating a first regression polynomial delimiting an upper line (103),
- based on the transitions delimiting the lower portion of the tear meniscus made up of the ends of segments corresponding to the transition from the fluorescent region to the non-fluorescent region, calculating a second regression polynomial delimiting a lower line (104).

9. The measuring method as claimed in claim 8, wherein calculating the regression polynomial uses the method of least squares.

10. The measuring method as claimed in claim 8 or 9, comprising calculating (260) the height (h) of the tear meniscus in pixels by calculating the distance between the regression polynomials calculated for each of said first line and second line down the columns of the image between the upper line and the lower line.

11. The measuring method as claimed in any one of the preceding claims, comprising selecting a region of interest (130) around the lower eyelid of said patient's eye in order to reduce calculation times and reduce detection errors.

12. The method as claimed in any one of the preceding claims, comprising a delay (35) suitable for waiting for resorption via the tear ducts of excess tear fluid comprising said fluorescein between the instillation of the fluorescein and the measurement.

13. A computer program comprising instructions for implementing the method as claimed in one of claims 1 to 12 when this program is executed by a processor.

14. A computer-readable non-volatile storage medium on which is stored a program for implementing the method as claimed in one of claims 1 to 12 when this program is executed by a processor.

15. An ophthalmic measuring device for implementing the method of any one of claims 1 to 12, **characterized in that** it comprises an ophthalmic measuring apparatus (1) provided with blue light sources (20) arranged around a lens (31) of at least one camera (30), a computer provided with a user interface (4, 5) and programmed to drive said sources and said at least one camera, and implement the method, said computer and said interface being integrated into the ophthalmic measuring device or being external thereto and connected to the ophthalmic measuring device.
